Europäisches Patentamt

European Patent Office

Office européen des brevets

(11) Publication number: **0 563 654 A1**

## EUROPEAN PATENT APPLICATION

(21) Application number: 93104063.8

(22) Date of filing: 12.03.93

(51) Int. Cl.5: **A61B 5/00, A61K 31/00**

(30) Priority: **18.03.92 US 853369**

(43) Date of publication of application:
**06.10.93 Bulletin 93/40**

(84) Designated Contracting States:
**DE FR GB**

(71) Applicant: **HOECHST-ROUSSEL
PHARMACEUTICALS INCORPORATED
P.O. Box 2500,
Route 202-206
Somerville, NJ 08876-1258(US)**

(72) Inventor: **Hardiman, Stephen
126 Vienna Hills Road
Hackettstown, New Jersey 07840(US)**
Inventor: **Miller, Kathryn
3 Abbott Road
Somerset, New Jersey 08873(US)**

(74) Representative: **Becker, Heinrich Karl
Engelbert, Dr. et al
HOECHST AKTIENGESELLSCHAFT,
Zentrale Patentabteilung,
Gebäude F 821
D-65926 Frankfurt am Main (DE)**

(54) **Risk determination for drug treatment.**

(57) This invention relates to a method for lowering the incidence of unwanted side effects associated with the treatment of a general population with a therapeutic agent known to have undesirable side effects. More particularly, this application relates to a method for using a statistical model to predict the probability of an occurrence for a particular individual within a patient population, then using the probability to determine the risk to the individual patient and refraining from treatment if the risk of developing an undesirable side effect is too great. Additionally, this invention relates to a method for obtaining the statistical model used in the process described above.

EP 0 563 654 A1

This invention relates to a method for lowering the incidence of unwanted side effects associated with the treatment of a general population with a therapeutic agent known to have undesirable side effects. More particularly, this application relates to a method for using a statistical model to predict the probability of an occurrence for a particular individual within a patient population, then using the probability to determine the risk to the individual patient and refraining from treatment if the risk of developing an undesirable side effect is too great. Additionally, this invention relates to a method for obtaining the statistical model used in the process described above.

Many drugs which are useful in the control of various diseases also have undesirable side effects. For example, about 20% to 30% of Alzheimer's Disease patients who are treated with a tetrahydroaminoacridine develop indications of liver toxicity as evidenced by the elevation of certain liver enzymes. While, for most patients, laboratory evidence of such hepatocellular injury disappears within about four weeks from the cessation of drug therapy, it would be advantageous to determine, before drug administration, which patients would have a greater risk of incurring such hepatotoxicity. A physician could then refrain from prescribing such a tetrahydroaminoacridine for a patient at high risk of developing hepatotoxicity.

Thus, it is an object of this invention to provide a method for lowering the incidence of unwanted side effects of drug therapy in a patient population.

More particularly, it is an object of this invention to provide a method for lowering the incidence of hepatocellular damage associated with the treatment of Alzheimer's patients with a tetrohydroaminoacridine.

It is also an object of this invention to provide a method for obtaining a statistical model which can be used in the determination of the probability of an individual within a patient population manifesting side effects to a given drug therapy.

In accordance with the present invention there is provided a method of lowering the incidence of undesirable side effects associated with a drug therapy in a patient population which comprises

obtaining demographic data and a plurality of clinical laboratory results for an individual within the patient population prior to treatment with a drug;

applying the obtained demographic data and clinical laboratory results to a statistical model to determine the probability of undesirable side effect for the individual;

evaluating the probability of the undesirable side effect in conjunction with the general health of the individual and determining the risk of treatment with the drug therapy; and

treating the individual where the risk is within medically accepted limits.

Further, in accordance with the invention there is provided a method for obtaining a statistical model which is effective to determine the probability of undesirable side effects of drug therapy for an individual, which method comprises;

gathering, prior to drug therapy, demographic data and a plurality of clinical laboratory results from individuals within a patient population wherein the clinical laboratory results are within the normal ranges for such results;

using logistic regression analysis to determine significant risk factors in the demographic data;

identifying predictive risk factors from the clinical laboratory results by entering the results into logistic regression to determine the significance of each risk factor for predicting the unwanted side effect;

combining the demographic risk factors with the laboratory result risk factors and determining the interdependence of all the risk factors;

identifying predictive risk factors from clinical laboratory risk factors, demographic risk factors and interactions between risk factors by entering each result into an individual logistic regression to determine the significance of each risk factor for predicting the unwanted side effects;

combining all the predictive risk factors into the same logistic regression and deleting any factor which substantially correlates with any other factor or any factor that does not predict the unwanted side effect; and

obtaining the statistical model.

Unless otherwise stated or indicated, the term "demographic data" means data such as, for example, age, gender, height, weight and genetic background.

Unless otherwise stated or indicated, the term "clinical laboratory results" means the results from blood level assays for hematology, serum chemistry, Vitamin E levels serum, Vitamin $B_{12}$ levels, folate levels and parameters measuring thyroid hormone levels.

Unless otherwise stated or indicated the term "undesirable side effect" means any physiological or psychological effect which threatens the individual's general health, for example, the hepatocellular damage which can arise following the treatment with tetrahydroaminoacridines.

Unless otherwise stated or indicated the term "hepatocellular damage" means any injury to the liver including but not limited to damage which is evidenced by at least one abnormal value greater than or

2

equal to twice the upper limit in serum glutamate pyruvate transaminase (SGPT) or serum glutamic-oxaloacetic transaminase (SGOT).

Unless otherwise stated or indicated, the term "medically acceptable limits" means those limits which are followed by the average professional working in the medical field or are set forth by a professional medical association.

Unless otherwise stated or indicated, the term "tetrahydroaminoacridine" means any tetrohydroaminoacridine derivative which is considered to have efficacy in the treatment of disease such as, for example, Alzheimer's disease, for example, 1,2,3,4-tetrahydro-9-acridinamine-1-ol (velnacrine maleate), 1,2,3,4-tetrahydro-9-acridinamine (tacrine), 9-phenylmethyl-1,2,3,4-tetrahydro-9-acridinamine-1-ol (suronacrine maleate), 7-methoxy-1,2,3,4-tetrahydro-9-acridinamine (7-methoxytacrine), and 8-fluoro-1,2,3,4-tetrahydro-2,4-methano-9-acridinamine) (SM-10888).

In a preferred embodiment of a process of this invention there is provided a method for lowering the incidence of hepatocellular damage associated with the treatment with an aminoacridine in Alzheimer's patients which comprises

obtaining demographic data and a plurality of clinical laboratory results for an individual Alzheimer patient prior to treatment with an aminoacridine;

applying the obtained demographic data and clinical laboratory results to a statistical model to determine the probability of hepatocellular damage for the individual Alzheimer's patient;

evaluating the probability of hepatocellular damage in conjunction with the general health of the individual and determining the risk to the individual of treatment with an aminoacridine;

treating the individual with an aminoacridine where the risk is within medically accepted limits and

effecting a lower incidence of hepatocellular damage in Alzheimer's patients treated with an aminoacridine.

More preferably, the demographic data is selected from age, gender, height, weight and ethnic origin; clinical laboratory results are selected from assay results for hematology, serum chemistry, Vitamin E levels, serum $B_{12}$ levels, folate levels and parameters measuring thyroid levels; the statistical model is a logistic regression wherein the probability P is defined as

$$ P = \frac{EXP(\hat{Y})}{1+EXP(\hat{Y})} $$

where $\hat{y}$ is the estimated linear regression of the predictors of hepatocellular damage expressed as

$$ \hat{Y} = \beta_0 + \Sigma \beta_k X_k $$

where $\beta_0$ is a statistically determined value, $\beta_k$ are statistically determined coefficients applied to the values for clinical laboratory results and demographic data, $X_k$ are values for clinical laboratory results and demographic data, and the aminoacridine is selected from the group of velnacrine maleate, tacrine, suronacrine maleate, 7-methoxytacrine and SM-10888.

Even more preferably, in one embodiment of the invention, the demographic data is age and gender; the clinical laboratory results are the values for folic acid, albumin, triglycerides and Vitamin $B_{12}$;

$$ \hat{Y} = \beta_0 + \beta_1{}^*GENDER + \beta_2{}^*AGE + \beta_3{}^*FOLIC\ ACID + \beta_4{}^*ALBUMIN + \beta_5{}^*TRIGLYCERIDES + \beta_6{}^*VITAMIN\ B_{12} + \beta_7{}^*AGE^*GENDER + \beta_8{}^*TRIGLYCERIDES^*GENDER $$

and the tetrahydroaminoacridine is velnacrine maleate.

Most preferably, in this emodiment $\hat{y}$ = 42.12680288 + (-15.46570582*GENDER) + (-0.01310740*AGE) + - (0.1513 3126*FOLIC ACID) + (9.85590207*ALBUMIN) + (0.00191341*TRIGLYCERIDES) + (-0.00377651 *VITAMIN B12) + (0.16957928*[AGE*GENDER]) + (0.03054193* [TRIGLYCERIDES*GENDER]); where GENDER is 0 for males and 1 for females, AGE is expressed in years, folic acid is expressed as nanograms per milliter (ng/ml), albumin is expressed as grams per deciliter (g/dl), triglycerides is expressed as milligrams per deciliter (mg/dl) and Vitamin $B_1$ is expressed as picograms per milliter (pg/ml).

More preferably, in another embodiment, the demographic data is age and gender; the clinical laboratory results are the values for platelets, white blood count (WBC), folic acid, blood urea nitrogen (BUN), T3 uptake, chloride, neutrophils (NEUTRO), SGPT and alkaline phosphatase (ALK PHOS);

$\hat{Y} = \beta_0 + \beta_1*\text{AGE} + \beta_2*\text{GENDER} + \beta_3*\text{PLATELET} + \beta_4*\text{WBC} + \beta_5*\text{FOLIC ACID} + \beta_6*\text{BUN} + \beta_7*\text{T3} + \beta_8*\text{CHLORIDE} + \beta_9*\text{NEUTRO} + \beta_{10}*\text{SGPT} + \beta_{11}*\text{ALKPHOS} + \beta_{12}*\text{BUN}*\text{AGE} + \beta_{13}*\text{PLATELET}*\text{GENDER} + \beta_{14}*\text{NEUTRO}*\text{GENDER} + \beta_{15}*\text{CHLORIDE}*\text{GENDER} + \beta_{16}*\text{SGPT}*\text{GENDER}$

and the tetrahydroaminoacridine is velnacrine maleate.

Most preferably, $\hat{y} = 45.72600017 + (-0.11554252*\text{AGE}) + (-35.92577279*\text{GENDER}) + - (0.01058219*\text{PLATELET}) + (-0.35364951*\text{WBC}) + (-0.03570996*\text{FOLIC ACID}) + (-0.3306*\text{BUN}) + (- 0.16604741*\text{T3}) + (-0.31021350*\text{CHLORIDE}) + (-0.06785345*\text{NEUTRO}) + (0.04837085*\text{SGPT}) + (0.00193039*\text{ALKPHOS}) + (0.00620017*[\text{BUN}*\text{AGE}]) + (0.00924488*[\text{PLATELET}*\text{GENDER}]) + (0.11422354*- [\text{NEUTRO}*\text{GENDER}]) + (0.32221264*[\text{CHLORIDE}*\text{GENDER}]) + (-0.10331888*[\text{SGPT}*\text{GENDER}])$ where age is expressed in years, gender is 1 for females and 0 for males, platelet is expressed as thousands per cubic millimeter (TH/CMM), WBC is expressed as thousands per cubic milliliter (TH/CMM), folic acid is expressed as nanograms per milliliter (ng/ml), BUN is expressed as milligrams per deciliter (mg/dl), T3 Uptake is expressed as percent (%), chloride is expressed as millimole per liter (mmol/l), neutrophils is expressed as percent (%), SGPT is expressed as International units per liter (IU/L), and alkaline phosphatase is expressed as International units per liter (IU/L).

In another preferred embodiment of the invention there is provided a method for obtaining a statistical model which is effective to determine the probability of hepatocellular damage associated with the treatment with an aminoacridine in Alzheimer's patients which comprises;

gathering, prior to treatment with the aminoacridine, demographic data and a plurality of clinical laboratory results from Alzheimer's patients where the clinical laboratory results are within the normal ranges for such results.

using logistic regression analysis to determine significant risk factors in the demographic data;

identifying predictive risk factors from the clinical laboratory results by entering the results into logistic regression to determine the significance of each risk factor for predicting hepatocellular damage;

combining the demographic risk factors with the laboratory result risk factors and determining all the risk factors;

identifying predictive risk factors from clinical laboratory risk factors, demographic risk factors and interactions between risk factors by entering each result into an individual logistic regression to determine the significance of each risk factor for predicting hepatocellular damage associated with the treatment with an acridine;

combining all the predictive risk factors into the same logistic regression and deleting any factor which substantially correlates with any other factor or any factor which does not predict hepatocellular damage; and

obtaining the statistical model.

More preferably, the demographic data was selected from age, gender, height, weight and ethnic origin; clinical laboratory results are selected from assay results for hematology serum chemistry, Vitamin E levels, serum $B_{12}$ levels, folate levels and parameters measuring thyroid levels; the statistical model is a logistic regression wherein the probability P is defined as

$$P = \frac{\text{EXP}(\hat{Y})}{1 + \text{EXP}(\hat{Y})}$$

where $\hat{y}$ is the estimated linear regression of the predictors of hepatocellular damage expressed as

$\hat{Y} = \beta_0 + \Sigma\beta_k X_k$

where $\beta_0$ is a statistically determined value, $\beta_k$ are statistically determined coefficients applied to the values for clinical laboratory results and demographic data, $X_k$ are values for clinical laboratory results and demographic data, and the aminoacridine is selected from the group of velnacrine maleate, tacrine, suronacrine maleate, 7-methoxytacrine and SM-10888.

Most preferably, in one embodiment of the invention, the demographic data is age and gender; the clinical laboratory results are the values for folic acid, albumin, triglycerides and Vitamin $B_{12}$;

$\hat{Y} = \beta_0 + \beta_1*\text{AGE} + \beta_2*\text{GENDER} + \beta_3*\text{PLATELET} + \beta_4*\text{WBC} + \beta_5*\text{FOLIC ACID} + \beta_6*\text{BUN} + \beta_7*\text{T3} +$

$\beta_8$*CHLORIDE + $\beta_9$*NEUTRO + $\beta_{10}$*SGPT + $\beta_{11}$*ALKPHOS + $\beta_{12}$*BUN*AGE + $\beta_{13}$*PLATELET*GENDER + $\beta_{14}$*NEUTRO*GENDER + $\beta_{15}$*CHLORIDE*GENDER + $\beta_{16}$*SGPT*GENDER

and the tetrahydroaminoacridine is velnacrine maleate.

Most preferably, in this emodiment $\hat{y}$ = 42.12680288 + (-15.46570582*GENDER) + (-0.01310740*AGE) + - (0.1513 3126*FOLIC ACID) + (9.85590207*ALBUMIN) + (0.00191341*TRIGLYCERIDES) + (-0.00377651 *VITAMIN B12) + (0.16957928*[AGE*GENDER]) + (0.03054193* [TRIGLYCERIDES*GENDER]); where GENDER is 0 for males and 1 for females, AGE is expressed in years, folic acid is expressed as nanograms per milliter (ng/ml), albumin is expressed as grams per deciliter (g/dl), triglycerides is expressed as milligrams per deciliter (mg/dl) and Vitamin $B_1$ is expressed as picograms per milliter (pg/ml).

More preferably, in another embodiment, the demographic data is age and gender; the clinical laboratory results are the values for platelets, white blood count (WBC), folic acid, blood urea nitrogen (BUN), T3 uptake, chloride, neutrophils (NEUTRO), SGPT and alkaline phosphatase (ALK PHOS);

$\hat{Y}$ = $\beta_0$ + $\beta_1$*AGE + $\beta_2$*GENDER + $\beta_3$*PLATELET + $\beta_4$*WBC + $\beta_5$*FOLIC ACID + $\beta_6$*BUN + $\beta_7$*T3 + $\beta_8$*CHLORIDE + $\beta_9$*NEUTRO + $\beta_{10}$*SGPT + $\beta_{11}$*ALKPHOS + $\beta_{12}$*BUN*AGE + $\beta_{13}$*PLATELET*GENDER + $\beta_{14}$*NEUTRO*GENDER + $\beta_{15}$*CHLORIDE*GENDER + $\beta_{16}$*SGPT*GENDER

and the tetrahydroaminoacridine is velnacrine maleate.

Most preferably, $\hat{y}$ = 45.72600017 + (-0.11554252*AGE) + (-35.92577279*GENDER) + - (0.01058219*PLATELET) + (-0.35364951*WBC) + (-0.03570996*FOLIC ACID) + (-0.3306*BUN) + (- 0.16604741*T3) + (-0.31021350*CHLORIDE) + (-0.06785345*NEUTRO) + (0.04837085*SGPT) + (0.00193039*ALKPHOS) + (0.00620017*[BUN*AGE]) + (0.00924488*[PLATELET*GENDER]) + (0.11422354*- [NEUTRO*GENDER]) + (0.32221264*[CHLORIDE*GENDER]) + (-0.10331888*[SGPT*GENDER]); wherein where age is expressed in years, gender is 1 for females and 0 for males, platelet is expressed as thousands per cubic millimeter (TH/CMM), WBC is expressed as thousands per cubic milliliter (TH/CMM), folic acid is expressed as nanograms per milliliter (ng/ml), BUN is expressed as milligrams per deciliter (mg/dl), T3 Uptake is expressed as percent (%), chloride is expressed as millimole per liter (mmol/l), neutrophils is expressed as percent (%), SGPT is expressed as International units per liter (IU/L), and alkaline phosphatase is expressed as International units per liter (IU/L).

Further, in accordance with this invention there is provided a device having a means for an operator to enter values for demographic data and predictive risk factors;

a means for using the values to calculate the probability of hepatocellular damage; and

a means for the operator to obtain the calculated values.

More preferably, there is provided a calculator programmed so that an operator can enter the found values for laboratory results and demographic data and obtain the probability of hepatocellular damage without additional calculations by the operator.

The statistical model of the invention is easy to use, reliable and completely determined by data gathered prior to patient treatment. The false negative rate is low and the specificity and sensitivity are high.

The theoretical basis for the statistical model was developed as follows:

The dependent variable y can be approximated by a model linear in x's, covariates. The data was written as a matrix in the form

$y_1$ $x_{11}$... $x_{21}$ ...$x_{k1}$

$y_2$ $x_{12}$... $x_{22}$ ...$x_{k2}$

$y_n$ $x_{1n}$... $x_{2n}$ ...$x_{kn}$

where n >k. The model which was assumed has the form

$$Y_i = \beta_0 + \beta_1 x_{li} + \beta_2 i + \ ... \ + \beta_k x_{ki} + \epsilon_i, \ (i = 1,2 \ ... \ n)$$

where $\epsilon_i$ is a random variable. This model is usually referred to as a regression equation.

It was assumed that the $\epsilon_i$ are independent with zero mean and variance $\sigma^2$. Thus, $E(\underline{\epsilon}) = 0$ and $Cov(\underline{\epsilon}) = \sigma^2 I$. In matrix notation

$$\underline{y} = X\hat{\underline{\beta}} + \underline{\epsilon}$$

The method of least squares is commonly used to estimate the parameters in the vector $\beta$. The least square estimators are unbiased with $Cov(\hat{\beta}) = \sigma^2 (X'X)^{-1}$.

Given the estimates $\hat{\beta}$, the estimated or predicted value of y was obtained for a given set of covariates. For any given set of covariates, the mean of the distribution of the dependent variables can be obtained.

The logistic model was used to relate disease probabilities to one or more covariate risk variables. This model allows the direct calculation, for a given set of covariates, of the probability of disease. Symbolically, the probability (P) of disease risk can be represented as the logit transform of y defined by

$$y = \text{logit } P = \log \left( \frac{P}{1 - P} \right)$$

or

$$P = \frac{\exp(y)}{1 + \exp(y)}$$

Note that P/(1-P) denotes the disease odds and the logit is sometimes called the log odds.

Assuming a set of k regression variables $(X_1 \ldots X_k)$, the theoretical probability (Pr) of disease given $X_i$, $i = 1, \ldots, k$ can be written by

$$\text{Pr}(y = 1/\underline{x}) = \frac{\exp(\beta_o + \Sigma \beta_k X_k)}{1 + \exp(\beta_o + \Sigma \beta_k X_k)}$$

or

$$\text{logit Pr}(y=1/\underline{x}) = \beta_o + \Sigma \beta_k X_k.$$

For individuals having two different sets of $\underline{x}$ and $\underline{x}^*$ of risk variables, the relative risk is given by

$$RR = \exp\{\Sigma \beta_k (x_k^* - x_k)\}$$

Thus, $\beta$ represents the log odds of disease risk for an individual with a standard set of regression variables, $\underline{x} = O$ while exp $(\beta_k)$ is the fraction by which the risk is increased (or decreased) for every change in $\bar{X}_k$.

The following examples are for illustration purposes only and are not to be construed as limiting the invention.

## EXAMPLE 1

A subset of fifty-four Alzheimer patients from a clinical trial testing the efficacy of velnacrine maleate therapy was studied. During the trial 19 of the 54 patients manifested hepatocellular damage by developing elevated values of SGPT or SGOT ($\geq$ 2 times the normal upper limit).

Prior to drug treatment, demographic data and values for laboratory assays for hematology, serum chemistry, Vitamin E levels, serum $B_{12}$ and folate levels as well as parameters measuring thyroid hormone levels were collected. Initially, using logistic regression both age and gender were identified significant risk factors, identifying elderly female patients as being at significantly high risk.

The means and standard deviation of each parameter was determined and, after removing those parameters which are clinically known to vary greatly in normal individuals, were used to choose a potential risk subset. With age and gender fixed in the model, each laboratory parameter was examined for model inclusion. Each of the potential risk variables was then entered into individual logistic equations to determine significance for predicting liver toxicity.

The following laboratory parameters were found to be potential predictors (risk variables): folic acid, albumin, triglycerides, cholesterol, Vitamin E and Vitamin $B_{12}$. Entering all the parameters simultaneously into the model, cholesterol was highly correlated with other variables and deleted.

The next question was whether the risk of any laboratory variable was possibly secondarily influenced by age or gender. The interactions showed that triglycerides and age were gender dependent.

The final model became

6

| Variable | Coefficient | P-Value |
|---|---|---|
| GENDER | -15.4657 | 0.168 |
| AGE | -0.0131 | 0.897 |
| FOLIC ACID | 0.1513 | 0.124 |
| ALBUMIN | 9.8559 | 0.008 |
| TRIGLYCERIDES | 0.0019 | 0.761 |
| VITAMIN $B_{12}$ | -0.0038 | 0.150 |
| TRIGEN | 0.0305 | 0.153 |
| AGEGEN | 0.1696 | 0.271 |

The model had 73% sensitivity (given the patients who developed disease, the percentage the model identified), 90% specificity (given the patients who were free of disease, the percentage the model identified) for a correct rate of 85%. The false negative rate was 13%. The model was given the name Physician References of Predicted Probabilities (PROPP).

**EXAMPLE 2**

A sixty year old male Alzheimer patient with folic acid (5.7 ng/ml), Vitamin $B_{12}$ (478 pg/ml), albumin (4.3 g/dl) and triglycerides (157 mg/dl) in general good health is a candidate for treatment with velnacrine maleate. Using PROPP, the predicted liver toxicity probability is calculated to be 23.6%. The physician prescribes the aminoacridine.

**EXAMPLE 3**

A sixty year old male Alzheimer patient with folic acid (5.7 ng/ml), Vitamin $B_{12}$ (478 pg/ml), albumin (4.3 g/dl) and triglycerides (157 mg/dl) in general poor weakened health is a candidate for treatment with velnacrine maleate. Using PROPP, the predicted liver toxicity probability is calculated to be 23.6%. The physician does not prescribe the aminoacridine.

**EXAMPLE 4**

A fifty-five year old female Alzheimer patient with folic acid (4.0 ng/ml), Vitamin $B_{12}$ (361 pg/ml), albumin (4.5 g/dl) and triglycerides (80 mg/dl) in general good health is a candidate for treatment with tacrine. The PROPP calculation shows a 5.8% probability of liver toxicity. The physician prescribes tacrine.

**EXAMPLE 5**

An eighty-two year old female with folic acid (13.7 ng/ml), Vitamin $B_{12}$ (399 pg/ml), albumin (4.3 g/dl) and triglycerides (163 mg/dl) is in general good health. The PROPP calculation shows a 97% probability of hepatotoxicity. The physician does not prescribe an aminoacridine.

**EXAMPLE 6**

A chart of relative risks enhances the application and utility of PROPP. The relative risk for two patients was calculated by

$$RR = \exp\{\Sigma\beta_k(X^+_k - X_k)\} = \frac{P(X^+)(1-P(X))}{P(X)(1-P(X^+))}$$

The relative risk provides a stable measure of association in a wide variety of human populations. Relative risk (a) compares the relative risk for gender within age level. As the patient's age increases there is an increase in risk for females with respect to males. Relative risk (b) compares each patient with sixty year old male. Even though the laboratory values vary, the relative risks for older patients, especially females, increase greatly.

## RELATIVE RISK FOR THE PROPP STATISTIC

| Age | Gender | Folic Acid | Vitamin B$_{12}$ | Albumin | Triglycerides | PROPP | Relative Risk[a] | Relative Risk[b] |
|---|---|---|---|---|---|---|---|---|
| 60 | Male | 5.7 | 478 | 4.3 | 157 | 23.6% | — | — |
| 60 | Female | 5.7 | 478 | 4.3 | 157 | 15.6% | 0.60 | 0.60 |
| 69 | Male | 11.9 | 498 | 4.1 | 126 | 3.9% | — | 0.13 |
| 69 | Female | 11.9 | 498 | 4.1 | 126 | 4.9% | 1.27 | 0.17 |
| 82 | Male | 13.7 | 399 | 4.3 | 163 | 51.1% | — | 3.38 |
| 82 | Female | 13.7 | 399 | 4.3 | 163 | 97.0% | 30.94 | 104.67 |

**EXAMPLE 7**

A subset of 197 Alzheimer patients from a clinical trial testing the efficacy of velnacridine maleate therapy was studied using a procedure substantially described in Example 1. Of the 197 patients, 69 had

8

liver elevation at 3 times the upper limit. The following laboratory parameters where found to be potential predictors (risk variables); age, gender, platelets, WBC, folic acid, BUN, T3 uptake, chloride, neutrophils, SGPT and alkaline phosphatase. Triglycerides and potassium were dropped due to a high correlation with other variables.

Based on the determination that BUN was modfied by age, and platelets, neutrophils, chloride and SGPT were modifed by gender, the final model became

| Variable | Coefficient | P-Value |
|---|---|---|
| Age | -0.1155 | 0.1989 |
| Gender | -35.9258 | 0.0146 |
| Platelets | 0.0106 | 0.0300 |
| WBC | -0.3536 | 0.0043 |
| Folic Acid | -0.0357 | 0.1093 |
| BUN | -0.3306 | 0.4021 |
| T3 Uptake | -0.1660 | 0.0100 |
| Chloride | -0.3102 | 0.0022 |
| Neutrophils | -0.0679 | 0.0433 |
| SGPT | 0.0484 | 0.1245 |
| Alkaline Phosphatase | 0.00193 | 0.7812 |
| BUNAge | 0.0062 | 0.2428 |
| PlatGen | -0.0092 | 0.1025 |
| NeuGen | 0.1142 | 0.0110 |
| ChlGen | 0.3222 | 0.0180 |
| SGPTGen | -0.1033 | 0.0162 |

The model has 49% sensitivity, 88% specificity for a correct rate of 75%. The positive predictive value is 69.4% and the negative predictive value is 76.4%.

## EXAMPLE 8

A sixty year old male Alzheimer patient with platelets (270 TH/CMM), WBC (7 TH/CMM), folic acid (14 ng/ml), BUN (15 mg/dl), T3 uptake (29%), chloride (91 mEq/l), neutrophils (63%), SGPT (19 IU/L) and alkaline phosphatase (97 IU/L) in general good health is a candidate for treatment with valacrine maleate. Using PROPP, the predicted liver toxicity probability is caculated to be 95.6%. The physician does not prescribe the aminoacridine.

## EXAMPLE 9

A sixty year old male Alzheimer patient with platelets (270 TH/CMM), WBC (7 TH/CMM), folic acid (14 ng/ml), BUN (15 mg/dl), T3 uptake (29%), chloride (108 mEq/l), neutrophils (63%), SGPT (19 IU/L) and alkaline phosphatase (97 IU/L) in general good health is a candidate for treatment with valacrine maleate. Using PROPP, the predicted liver toxicity probability is caculated to be 10.1%. The physician prescribes the aminoacridine.

## EXAMPLE 10

A sixty year old male Alzheimer patient with platelets (270 TH/CMM), WBC (7 TH/CMM), folic acid (14 ng/ml), BUN (15 mg/dl), T3 uptake (19%), chloride (102 mEq/l), neutrophils (63%), SGPT (19 IU/L) and alkaline phosphatase (97 IU/L) in general poor health is a candidate for treatment with valacrine maleate. Using PROPP, the predicted liver toxicity probability is caculated to be 79.1%. The physician does not prescribe the aminoacridine.

## EXAMPLE 11

A sixty year old female Alzheimer patient with platelets (270 TH/CMM), WBC (7 TH/CMM), folic acid (14 ng/ml), BUN (15 mg/dl), T3 uptake (39%), chloride (102 mEq/l), neutrophils (63%), SGPT (19 IU/L) and alkaline phosphatase (97 IU/L) in general good health is a candidate for treatment with valacrine maleate.

Using PROPP, the predicted liver toxicity probability is caculated to be 9.0%. The physician prescribes the aminoacridine.

**EXAMPLE 12**

A sixty year old male Alzheimer patient with platelets (270 TH/CMM), WBC (3 TH/CMM), folic acid (14 ng/ml), BUN (15 mg/dl), T3 uptake (29%), chloride (102 mEq/l), neutrophils (63%), SGPT (19 IU/L) and alkaline phosphatase (97 IU/L) in general good health is a candidate for treatment with valacrine maleate. Using PROPP, the predicted liver toxicity probability is caculated to be 74.7%. The physician does not prescribe the aminoacridine.

**EXAMPLE 13**

A sixty year old female Alzheimer patient with platelets (270 TH/CMM), WBC (13 TH/CMM), folic acid (14 ng/ml), BUN (15 mg/dl), T3 uptake (29%), chloride (102 mEq/l), neutrophils (63%), SGPT (19 IU/L) and alkaline phosphatase (97 IU/L) in general good health is a candidate for treatment with valacrine maleate. Using PROPP, the predicted liver toxicity probability is caculated to be 5.8%. The physician prescribes the aminoacridine.

It should be understood that the instant specification and examples are set forth by way of illustration and not limitation, and that various modifications and changes may be made without departing from the spirit and scope of the present invention as defined by the appended claims.

**Claims**

1. A method of lowering the incidence of undesirable side effects associated with a drug therapy in a patient population which comprises;

   obtaining demographic data and a plurality of clinical laboratory results for an individual within the patient population to treatment with a drug;

   applying the obtained demographic data and clinical laboratory results to a statistical model to determine the probability of undesirable side effect for the individual;

   correlating the probability of the undesirable side effect with the individuals' general health and determining the risk of treatment with the drug therapy and

   treating the individual where the risk is within medically accepted limits.

2. The method of Claim 1, for lowering the incidence of hepatocellular damage associated with the treatment with an aminoacridine in Alzheimer's patients which comprises;

   obtaining demographic data and a plurality of clinical laboratory results for an individual Alzheimer patient prior to treatment with an aminoacridine;

   applying the obtained demographic data and clinical laboratory results to a statistical model to determine the probability of hepatocellular damage for the individual Alzheimer's patient;

   evaluating the probability of hepatocellular damage in conjunction with the general health of the individual and determining the risk to the individual of treatment with an aminoacridine;

   treating the individual with an aminoacridine where the risk is within medically accepted limits and

   effecting a lower incidence of hepatocellular damage in Alzheimer's patients treated with an aminoacridine.

3. The method of Claim 2 wherein the demographic data is selected from age, gender, height, weight and ethnic origin; clinical laboratory results are selected from assay results for hematology, serum chemistry, Vitamin E levels, serum $B_{12}$ levels, folate levels and parameters measuring thyroid levels; the statistical model is a logistic regression wherein the probability P is defined as

$$P = \frac{EXP(\hat{Y})}{1+EXP(\hat{Y})}$$

where $\hat{y}$ is the estimated linear regression of the predictors of hepatocellular damage expressed as

$$\hat{Y} = \beta_0 + \Sigma\beta_k X_k$$

where $\beta_0$ is a statistically determined value, $\beta_k$ are statistically determined coefficients applied to the values for clinical laboratory results and demographic data, $X_k$ are values for clinical laboratory results and demographic data and the aminoacridine is selected from the group of velnacrine maleate, tacrine, suronacrine maleate, 7-methoxytacrine and SM-10888.

4. The method of Claim 3 wherein the demographic data are age and gender; the clinical laboratory results are the values for folic acid, albumin, triglycerides and Vitamin $B_{12}$;

$$\hat{Y} = \beta_0 = \beta_1\text{*GENDER} + \beta_2\text{*AGE} + \beta_3\text{*FOLIC ACID} + \beta_4\text{*ALBUMIN} + \beta_5\text{*TRIGLYCERIDES} + \beta_6\text{*VITAMIN } B_{12} + \beta_7\text{*AGE*GENDER} + \beta_8\text{*TRIGLYCERIDES*GENDER}$$

and the tetrahydroaminoacridine is velnacrine maleate.

5. The method of Claim 4 wherein $\hat{y}$ = 42.12680288 + (-15.46570582*GENDER) + (-0.01310740*AGE) + - (0.1513 3126*FOLIC ACID) + (9.85590207*ALBUMIN) + (0.00191341*TRIGLYCERIDES) + (-0.00377651 *VITAMIN B12) + (0.16957928*[AGE*GENDER]) + (0.03054193* [TRIGLYCERIDES*GENDER]); where GENDER is 0 for males and 1 for females, AGE is expressed in years, folic acid is expressed as nanograms per milliter (ng/ml), albumin is expressed as grams per deciliter (g/dl), triglycerides is expressed as milligrams per deciliter (mg/dl) and Vitamin $B_1$ is expressed as picograms per milliter (pg/ml).

6. The method of Claim 3 wherein the demographic data are age and gender; the clinical laboratory results are the values for platelets, white blood count (WBC), folic acid, blood urea nitrogen (BUN), T3 uptake, chloride, neutrophils (NEUTRO), SGPT and alkaline phosphatase (ALK PHOS);

$$\hat{Y} = \beta_0 + \beta_1\text{*AGE} + \beta_2\text{*GENDER} + \beta_3\text{*PLATELET} + \beta_4\text{*WBC} + \beta_5\text{*FOLIC ACID} + \beta_6\text{*BUN} + \beta_7\text{*T3} + \beta_8\text{*CHLORIDE} + \beta_9\text{*NEUTRO} + \beta_{10}\text{*SGPT} + \beta_{11}\text{*ALKPHOS} + \beta_{12}\text{*BUN*AGE} + \beta_{13}\text{*PLATELET*GENDER} + \beta_{14}\text{*NEUTRO*GENDER } \beta_{15}\text{*CHLORIDE*GENDER} + \beta_{16}\text{*SGPT*GENDER}$$

and the tetrahydroaminoacridine is velnacrine maleate.

7. The method of Claim 6 wherein $\hat{y}$ = 45.72600017 + (-0.11554252*AGE) + (-35.92577279*GENDER) + - (0.01058219*PLATELET) + (-0.35364951*WBC) + (-0.03570996*FOLIC ACID) + (-0.3306*BUN) + (- 0.16604741*T3) + (-0.31021350*CHLORIDE) + (-0.06785345*NEUTRO) + (0.04837085*SGPT) + (0.00193039*ALKPHOS) + (0.00620017*[BUN*AGE]) + (0.00924488*[PLATELET*GENDER]) + - (0.11422354*[NEUTRO*GENDER]) + (0.32221264*[CHLORIDE*GENDER]) + (-0.10331888*- [SGPT*GENDER]); where age is expressed in years, gender is 1 for females and 0 for males, platelet is expressed as thousands per cubic millimeter (TH/CMM), WBC is expressed as thousands per cubic milliliter (TH/CMM), folic acid is expressed as nanograms per milliliter (ng/ml), BUN is expressed as milligrams per deciliter (mg/dl), T3 Uptake is expressed as percent (%), chloride is expressed as millimole per liter (mmol/l), neutrophils is expressed as percent (%), SGPT is expressed as International units per liter (IU/L), and alkaline phosphatase is expressed as International units per liter (IU/L).

8. A method for obtaining a statistical model which is effective to determine the probability of undesirable side effects of drug therapy for an individual, which method comprises

gathering, prior to drug therapy, demographic data and a plurality of clinical laboratory results from individuals from within a patient population wherein the clinical laboratory results are within the normal ranges for such results;

using logistic regression analysis to determine significant risk factors in the demographic data;

identifying predictive risk factors from the clinical laboratory results by entering the results into individual logistic regression to determine the significance of each risk factor for predicting the unwanted side effect;

combining the demographic risk factors with the laboratory result risk factors and determining the interdependence of all the risk factors;

identifying predictive risk factors from clinical laboratory risk factors and demographic risk factors and interactions between risk factors by entering each result into an individual logistic regression to determine the significance of each risk factor for predicting the unwanted side effects;

combining all the predictive risk factors into the same logistic regression and deleting any factor which substantially correlates with any other factor or any factor that does not predict the unwanted side effect; and

obtaining the statistical model.

9. A method for obtaining a statistical model which is effective to determine the probability of hepatocellular damage associated with the treatment with an aminoacridine in Alzheimer's patients which comprises

gathering, prior to treatment with the aminoacridine, demographic data and a plurality of ctinical laboratory results from Alzheimer's patients where the clinical laboratory results are within the normal ranges for such results.

using logistic regression analysis to determine significant risk factors in the demographic data;

identifying predictive risk factors from the clinical laboratory results by entering each result in an individual logistic regression to determine the significance of each risk factor for predicting hepatocellular damage;

combining the demographic risk factors with the laboratory result risk factors and determining all the risk factors;

identifying predictive risk factors from clinical laboratory risk factors, demographic risk factors and interactions between risk factors by entering each result into an individual logistic regression to determine the significance of each risk factor for predicting hepatocellular damage associated with the treatment with an acridine;

entering all the predictive risk factors from the laboratory results into the same logistic regression and deleting any factor which substantially correlates with any other risk factors; and

obtaining the statistical model.

10. The method of Claim 9, wherein the demographic data are selected from age, gender, height, weight, and ethnic origin; clinical laboratory results are selected from assay results for hematology, serum chemistry, Vitamin E levels, serum $B_{12}$ levels, folate levels and parameters measuring thyroid levels; the statistical model is a logistic regression wherein the probability P is defined as

$$ P = \frac{EXP(\hat{Y})}{1+EXP(\hat{Y})} $$

where $\hat{y}$ is the estimated linear regression of the predictors of hepatocellular damage expressed as

$$ \hat{Y} = \beta_0 + \Sigma \beta_k X_k $$

where $\beta_0$ is a statistically determined value, $\beta_k$ are statistically determined coefficients applied to the values for clinical laboratory results and demographic data, $X_k$ are values for clinical laboratory results and demographic data and the aminoacridine is selected from the group of velnacrine maleate, tacrine, suronacrine maleate, 7-methoxytacrine and SM-10888.

11. The method of Claim 10 wherein the demographic data are age and gender; the clinical laboratory results are the values for folic acid, albumin, triglycerides and Vitamin $B_{12}$;

$$ \hat{Y} = \beta_0 + \beta_1*GENDER + \beta_2*AGE + \beta_3*FOLIC\ ACID + \beta_4*ALBUMIN + \beta_5*TRIGLYCERIDES + \beta_6*VITAMIN\ B_{12} + \beta_7*AGE*GENDER + \beta_8*TRIGLYCERIDES*GENDER $$

and the aminoacridine is velnacrine maleate.

12. The method of Claim 11 wherein $\hat{y}$ = 42.12680288 + (-15.46570582*GENDER) + (-0.01310740*AGE) + - (0.1513 3126*FOLIC ACID) + (9.85590207*ALBUMIN) + (0.00191341*TRIGLYCERIDES) + (-0.00377651 *VITAMIN B12) + (0.16957928*[AGE*GENDER]) + (0.03054193* [TRIGLYCERIDES*GENDER]); where GENDER is 0 for males and 1 for females, AGE is expressed in years, folic acid is expressed as nanograms per milliliter (ng/ml), albumin is expressed as grams per deciliter (g/dl), triglycerides is expressed as milligrams per deciliter (mg/dl) and Vitamin $B_1$ is expressed as picograms per milliliter (pg/ml).

**13.** The method of Claim 10 wherein the demographic data are age and gender; the clinical laboratory results are the values for platelets, white blood count (WBC), folic acid, blood urea nitrogen (BUN), T3 uptake, chloride, neutrophils (NEUTRO), SGPT and alkaline phosphatase (ALK PHOS);

$\hat{Y} = \beta_0 + \beta_1*AGE + \beta_2*GENDER + \beta_3*PLATELET + \beta_4*WBC + \beta_5*FOLIC\ ACID + \beta_6*BUN + \beta_7*T3 + \beta_8*CHLORIDE + \beta_9*NEUTRO + \beta_{10}*SGPT + \beta_{11}*ALKPHOS + \beta_{12}*BUN*AGE + \beta_{13}*PLATELET*GENDER + \beta_{14}*NEUTRO*GENDER\ \beta_{15}*CHLORIDE*GENDER + \beta_{16}*SGPT*GENDER$

and the aminoacridine is velnacrine maleate.

**14.** The method of Claim 13 wherein $\hat{y}$ = 45.72600017 + (-0.11554252*AGE) + (-35.92577279*GENDER) + -(0.01058219*PLATELET) + (-0.35364951*WBC) + (-0.03570996*FOLIC ACID) + (-0.3306*BUN) + (-0.16604741*T3) + (-0.31021350*CHLORIDE) + (-0.06785345*NEUTRO) + (0.04837085*SGPT)- + (0.00193039*ALKPHOS) + (0.00620017*[BUN*AGE]) + (0.00924488*[PLATELET*GENDER]) + -(0.11422354*[NEUTRO*GENDER]) + (0.32221264*[CHLORIDE*GENDER]) + (-0.10331888*-[SGPT*GENDER]) where age is expressed in years, gender is 1 for females and 0 for males, platelet is expressed as thousands per cubic millimeter (TH/CMM), WBC is expressed as thousands per cubic milliliter (TH/CMM), folic acid is expressed as nanograms per milliliter (ng/ml), BUN is expressed as milligrams per deciliter (mg/dl), T3 Uptake is expressed as percent (%), chloride is expressed as millimole per liter (mmol/l), neutrophils is expressed as percent (%), SGPT is expressed as International units per liter (IU/L), and alkaline phosphatase is expressed as International units per liter (IU/L).

**European Patent Office**

**DECLARATION** which under Rule 45 of the European Patent Convention shall be considered, for the purpose of subsequent proceedings, as the European search report

Application number

93104063

| The Search Division considers that the present European patent application does not comply with the provisions of the European Patent Convention to such an extent that it is not possible to carry out a meaningful search into the state of the art on the basis of all claims.<br>Reason: | CLASSIFICATION OF THE APPLICATION (Int. Cl.⁵) |
|---|---|
| The application concerns a method of evaluation of a therapeutical risk with a mathematical formula and should not be considered as patentable according to EPC (articles 52 and 57). | A 61 B 5/00<br>A 61 B 31/00 |

| Place of search | Date of completion of the search | Examiner |
|---|---|---|
| Berlin | 19-04-1993 | Avedikian P. |

EPO Form 1504 06.78